# EUROPÄISCHE PATENTSCHRIFT

(11) **EP 1 230 906 B1**
(45) Veröffentlichungstag und Bekanntmachung des Hinweises auf die Patenterteilung: **22.09.2004**
(21) Anmeldenummer: 02002077.2
(22) Anmeldetag: 11.02.2002
(51) Int. Cl.: A61K 6/02, A61K 6/06, A61K 6/083

(54) **Thermochromer Dentalwerkstoff**
Thermochromic dental material
Matériau dentaire thermochromique

(30) Priorität: 12.02.2001 DE 10106372
(43) Veröffentlichungstag der Anmeldung: 14.08.2002
(73) Patentinhaber: Ivoclar Vivadent AG, 9494 Schaan (LI)
(72) Erfinder: Burgath, Armin, 78351 Bodman-Ludwigshafen (DE); Burtscher, Peter, A-6830 Rankweil (AT); Salz, Ulrich, 88131 Lindau (DE); Rheinberger, Volker, FL-9490 Vaduz (LI)
(74) Vertreter: UEXKÜLL & STOLBERG

(56) Entgegenhaltungen:
- EP-A- 0 389 239
- EP-A- 0 610 072
- WO-A-92/00056
- DE-A- 3 939 998
- US-A- 4 600 389
- US-A- 5 698 020

## Beschreibung

Die Erfindung betrifft Dentalwerkstoffe, die einen thermochromen Farbstoff enthalten und die bei Temperaturänderungen eine reversible Farbänderung zeigen.

In der restaurativen Zahnheilkunde werden aus ästhetischen Gründen in zunehmendem Maße zahnfarbene Restaurationswerkstoffe eingesetzt. Diese Werkstoffe haben den Nachteil, daß sie sich visuell nur schwer von der natürlichen Zahnsubstanz unterscheiden lassen, so daß das Entfernen von überschüssigem Material sowie die Nachbearbeitung und Anpassung beispielsweise von Füllungen erschwert wird. Das hat zur Folge, daß häufig unnötigerweise gesunde Zahnsubstanz entfernt oder andererseits überschüssiger Dentalwerkstoff übersehen wird, welcher dann als Retentionsnische die Bildung von Plaque begünstigen und zu Parodontalproblemen führen kann. Auch bei der Entfernung zahnfarbener Füllungen wird wegen der schlechten Sichtbarkeit des Übergangs zwischen Füllung und Zahnsubstanz oft entweder zu viel gesunde Zahnsubstanz entfernt oder aber Reste der Füllung werden übersehen. Ähnliche Probleme ergeben sich bei der Verwendung von zahnfarbenen Befestigungsmaterialien zur Zementierung von zahnfarbenen Restaurationen.

Die EP 0 610 072 A2 betrifft Zahnbürsten, die ein thermochromes Material enthalten und die beim Gebrauch durch Verfärben die Putzzeit anzeigen.

Die US 4,957,949 offenbart Masterbatches, die Wachs und ein darin homogen dispergiertes, granuläres thermochromes Material enthalten, das mit einer hochmolekularen, hydrophilen Substanz beschichtet ist. Die Masterbatches sollen sich auch bei hohen Temperaturen und Drücken ohne Verlust der thermochromen Eigenschaften in thermoplastische Kunststoffe einarbeiten lassen. Die farbstoffhaltigen thermoplastischen Materialien sollen sich besonders zur Herstellung von Zahnbürsten eignen.

Die US 5,431,697 offenbart Zusammensetzungen auf der Basis von Olefinpolymeren, welche thermochrome Farbstoffe enthalten. Die Farbstoffe werden vorzugsweise in einer granulären Form eingesetzt, die durch Mischen von Polymer und Farbstoff und anschließendes Vernetzen des Polymers erhältlich ist. Das erhaltene Granulat soll sich insbesondere zum Mischen mit thermoplastischen Kunststoffen eignen, die dann zu sich verfärbenden Zahnbürsten weiterverarbeitet werden können.

Die US 3,619,254 offenbart mehrschichtige Artikel, die neben einer Basisschicht und mindestens einer Schutzschicht eine thermochrome Schicht aufweisen, die flüssigkristalline, thermochrome Farbstoffe enthält. Die Artikel sollen sich in Form rechteckiger Streifen zur Bestimmung der Körpertemperatur eignen.

Die US 4,022,706 offenbart thermochrome, flüssigkristalline Druckfarben in Form von viskosen Öl-in-Wasser-Emulsionen, die sich insbesondere zum Bedrucken von Folien und Laminaten eignen sollen. Die Druckfarben werden auf die Folien aufgebracht und nicht in diese eingearbeitet.

In der US 5,162,130 werden Dentalwerkstoffe offenbart, deren Färbung durch Bestrahlen mit UV-Licht und anschließendes Erhitzen eingestellt werden kann. Die Materialien werden hierbei permanent verfärbt und eignen sich nicht zur vorübergehenden Sichtbarmachung von farblosen oder zahnfarbenen Dentalwerkstoffen.

Die DE 195 02 751 A1 offenbart ein Verfahren zur Herstellung von Kunststoffmodellen für die Zahntechnik, die sich durch eine kontrastreiche, farbige Oberfläche auszeichnen. Dies wird durch Integration beispielsweise eines thermochromen Farbstoffs in die Oberfläche des Modells erreicht.

Die US 5,698,020 offenbart Dentalmaterialien, die neben ethylenisch ungesättigtem Monomer und Polymerisationsinitiator photochromen Farbstoff enthalten.

Dentalmaterialien, die einen thermochromen Farbstoff enthalten, sind bisher nicht bekannt.

Aufgabe der vorliegenden Erfindung ist die Schaffung eines Dentalwerkstoffs, dessen Farbe sich auf einfache Weise vorübergehend so verändern läßt, daß der Werkstoff von der natürlichen Zahnsubstanz visuell unterscheidbar ist, und der nach einem zur Bearbeitung des Dentalwerkstoffs ausreichenden Zeitraum wieder seine ursprüngliche Farbe annimmt.

Diese Aufgabe wird durch Dentalwerkstoffe gelöst, welche neben mindestens einem polymerisierbaren ethylenisch ungesättigten Monomer und mindestens einem Initiator für die Kalt-, Heißund/oder Photopolymerisation mindestens einen thermochromen Farbstoff enthalten, der in Abhängigkeit von der Temperatur seine Farbe reversibel ändert.

Unter Dentalwerkstoffen werden Materialien verstanden, die zur Anwendung im Munde des Patienten geeignet sind, d.h. beispielsweise als Dentalrestauration, als Bestandteil einer Dentalrestauration oder zur Befestigung einer Dentalrestauration oder orthopädischen Vorrichtung im Munde des Patienten dienen.

Unter thermochromen Farbstoffen werden anorganische oder vorzugsweise organische Substanzen verstanden, die in Abhängigkeit von der Temperatur ihre Farbe reversibel ändern.

Erfindungsgemäß sind solche thermochromen Farbstoffe bevorzugt, die bei einer Temperatur von etwa 37°C farblos sind und die sich beim Erwärmen oder vorzugsweise Abkühlen verfärben, d.h. eine von der natürlichen Zahnsubstanz deutlich unterscheidbare Farbe annehmen. Bei einer Temperatur von etwa 37 °C wird damit die Farbe des Dentalwerkstoffs durch dessen Eigenfarbe bestimmt.

Besonders bevorzugt sind solche thermochromen Farbstoffe, die sich bei einer Temperatur 29 °C oder weniger, vorzugsweise von 5 bis 29 °C, insbesondere 12 bis 29 °C und ganz besonders bevorzugt 20 bis 29 °C verfärben, oder Materialien, die sich bei Temperaturen von 40 °C oder mehr, vorzugsweise 40 bis 60 °C und insbesondere 45 bis 55 °C verfärben.

Dabei sind weiterhin solche Farbstoffe bevorzugt, die eine rote, blaue oder schwarze Färbung annehmen und sich damit besonders gut von der natürlichen Zahnsubstanz unterscheiden lassen.

Bevorzugt sind thermochrome Farbstoffe, die einen Elektronendonator und einen Elektronenakzeptor enthalten, oder Farbstoffe, die eine säurereaktive Komponente und eine Säurekomponente enthalten.

Als thermochrome Farbstoffe auf der Basis eines Elektronendonators und Elektronenakzeptors eignen sich besonders Mischungen aus einem Elektronen abgebenden Chromogen (Elektronendonator) und einem Elektronenakzeptor. Als Elektronendonatoren sind substituierte Phenylmethane, Fluorane, wie beipsielsweise 3,3'-Dimethoxyfluoran, 3-Chlor-6-phenylaminofluoran, 3-Diethylamino-6-methyl-7-chlorfluoran, 3-Diethyl-7,8-benzofluoran, 3,3',3"-Tris(p-di-methylaminophenyl)phthalid, 3,3'-Bis(p-dimethylaminophenyl)-7-phenylaminofluoran und 3-Diethylamino-6-methyl-7-phenylaminofluoran, Indolylphthalide, Spiropyrane und Cumarine sowie Mischungen dieser Substanzen bevorzugt.

Als Elektronenakzeptoren eignen sich besonders Phenole, Azole, organische Säuren und Ester sowie Salze organischer Säuren. Beispielhafte Phenole sind Phenylphenol, Bisphenol A, Cresol, Resorcinol, Chlorlucinol, Phenol, Phenololigomere, β-Naphthol, 1,5-Dihydroxynaphthalin, Pyrocatechol, Pyrogallol, und das Trimer von p-Chlorphenolformaldehydkondensat. Beispielhafte Azole sind Benzotriazole, wie 5-Chlorbenzotriazol, 4-Laurylaminosulfobenzotriazol, 5-Butylbenzotriazol, Dibenzotriazol, 2-Oxybenzotriazol, 5-Ethoxycarbonylbenzotriazol, 5,5'-Methylenbisbenzotriazol, Imidazole, wie Oxybenzimidazol, und Tetrazole. Die organischen Säuren umfassen beispielsweise aromatische und aliphatische Carbonsäuren und substituierte Derivate davon. Beispiele für aromatische Carbonsäuren sind Salicylsäure, Methylenbissalicylsäure, β-Resorcylsäure, Gallussäure, Benzoesäure, p-Oxybenzoesäure, Pyromellitsäure, β-Naphthoesäure, Gerbsäure, Toluylsäure, Trimellithsäure, Phthalsäure, Terephthalsäure und Anthranilsäure. Beispiele für aliphatische Carbonsäuren sind Säuren mit 1 bis 20 Kohlenstoffatomen, vorzugsweise 3 bis 15 Kohlenstoffatomen, wie beispielsweise Stearinsäure, 1,2-Hydroxystearinsäure, Weinsäure, Zitronensäure, Oxalsäure und Laurinsäure. Beispiele für Ester sind Alkylester aromatischer Carbonsäuren in denen der Alkylrest 1 bis 6 Kohlenstoffatome aufweist, wie Butylgallat, Ethyl-phydroxybenzoat und Methylsalicylat. Beispielhafte Salze sind Ammonium- und Metallsalze der oben genannten Säuren. Die Metallsalze umfassen zum Beispiel Lithium-, Natrium-, Calcium-, Magnesium-, Aluminium-, Zink-, Zinn-, Titan- und Nickelsalze. Besonders bevorzugte Elektronenakzeptoren sind 1,2-Hydroxystearinsäure, Weinsäure und Zitronensäure. Die oben genannten Elektronenakzeptoren können allein oder in Mischung miteinander eingesetzt werden. Weiterhin können die thermochromen Materialien als solche oder in mikroverkapselter Form verwendet werden. Geeignete Farbstoffe dieses Typs werden in der US 4,957,949 beschrieben.

Als thermochrome Farbstoffe auf der Basis einer säurereaktiven Komponente und eine Säurekomponente sind Mischungen aus einer säurereaktiven chromogenen Substanz und einer aciden Substanz (Säurekomponente) bevorzugt.

Bevorzugte säurereaktive Substanzen sind Triphenylmethanphthalide, Phthalide, Phthalane, Acylleucomethylenblauverbindungen, Fluorane, Triphenylmethane, Diphenylmethane, Spiropyrane und Derivate dieser Substanzen. Beispielhafte Verbindungen sind 3,6-Dimethoxyfluoran, 3,6-Dibutoxyfluoran, 3-Diethylamino-6,8-dimethylfluoran, 3-Chlor-6-phenylaminofluoran, 3-Diethylamino-6-methyl-7-chlorfluoran, 3-Diethylamino-7,8-benzofluoran, 2-Anilino-3-methyl-6-diethylaminofluoran, 3,3',3"-Tris(p-dimethylaminophenyl)phthalid, 3,3'-Bis(p-dimethylaminophenyl)phthalid, 3-Diethylamino-7-phenylaminofluoran, 3,3-Bis(p-diethylaminophenyl)-6-dimethylaminophthalid,3-(4-Diethylaminophenyl)-3-(1-ethyl-2-methylindol-3-yl)phthalid, 3-(4-Diethylamino-2-methyl)phenyl-3-(1,2-dimethylindol-3-yl)phthalid und 2'-(2-Chloranilino)-6'-dibutylaminospiro-[phthalido-3,9'-xanthen].

Bevorzugte acide Substanzen sind 1,2,3-Benzotriazole, Phenole, Thioharnstoffe, oxoaromatische Carbonsäuren und Derivate dieser Substanzen. Beispielhafte Verbindungen sind 5-Butylbenzotriazol, Bisbenzotriazol-5-methan, Phenol, Nonylphenol, Bisphenol A, Bisphenol F, 2,2'-Biphenol, β-Naphthol, 1,5-Dihydroxynaphthalin, Alkyl-p-hydroxybenzoate und Phenolharzoligomere. Diese Farbstoffe können ebenfalls als solche oder in mikroverkapselter Form eingesetzt werden. Geeignete Farbstoffe diesen Typs werden in der US 5,431,697 beschrieben und können von den Firmen Dainichiseika Color & Chemicals Co., Ltd., beispielsweise unter der Bezeichnung Yellow PP-020", von Hodogaya Chemical Co., Ltd., oder von Matsui Shikiso Chemical Co., Ltd., beispielsweise unter den Bezeichnungen Photopia Yellow" oder Chromicolor Fast Blue S-17", bezogen werden.

Weiter bevorzugte thermochrome Farbstoffe sind flüssigkristalline Cholesterinderivate, wie Alkansäure- und Aralkansäureester des Cholesterins, Alkylester von Cholesterincarbonat und Mischungen davon, insbesondere solche mit Alkyl- und Alkansäuregruppen mit 1 bis 24 Kohlenstoffatomen. Cholesterinester und Derivate davon, die eine Alkansäuregruppe mit 9 bis 22 Kohlenstoffatomen oder eine Aralkansäuregruppe mit einer Benzoesäuregruppe und 1 bis 3 Kohlenstoffatomem im Alkylteil enthalten, sind besonders bevorzugt. Im Fall der Cholesterincarbonatester sind solche mit C₁- bis C₂₀-Alkylgruppen bevorzugt. Geeignete Verbindungen dieses Typs werden in der US 3,619,254 beschrieben.

Weitere bevorzugte flüssigkristalline Cholesterinderivate sind Cholesterinchlorid, Cholesterinbromid, Cholesterinacetat, Cholesterinoleat, Cholesterincaprylat, Cholesterinoleylcarbonat, Mischungen davon und Mischungen dieser Farbstoffe mit den zuvor genannten Cholesterinderivaten. Diese und weitere Farbstoffe werden in der US 4,022,706 und der US 3,600,060 beschrieben. Geeignete Farbstoffe können von der Firma Davis Liquid Crystals, Inc., USA unter der Bezeichnung Chromazone" bezogen werden.

Thermochrome Farbstoffe werden vorzugsweise in einer Menge von 0,01 bis 2 Gew.-%, besonders bevorzugt 0,1 bis 0,5 Gew.-% und ganz besonders bevorzugt in einer Menge von etwa 0,2 Gew.-% bezogen auf die Gesamtmasse des Dentalwerkstoffs eingesetzt.

Die erfindungsgemäßen Dentalmaterialien sind bei Körpertemperatur, d.h. bei etwa 37 °C, farblos, d.h. nicht gefärbt, und können durch Erwärmen oder vorzugsweise durch Abkühlen verfärbt werden. Dies kann beispielweise dadurch erfolgen, daß der Patient vor oder während einer Behandlung durch den Zahnarzt mit einer kalten oder warmen Flüssigkeit spült oder indem der Zahnarzt den betreffenden Zahn mit einem Luftstrahl kühlt. Das gefärbte Dentalmaterial läßt sich anschließend gut von der natürlichen Zahnsubstanz unterscheiden und kann so vom Zahnarzt gezielt bearbeitet werden.

Neben dem thermochromen Material enthalten die erfindungsgemäßen Dentalwerkstoffe mindestens ein ethylenisch ungesättigtes Monomer als Bindemittel und mindestens einen Initiator für die Heiß-, Kalt- oder vorzugsweise die Photopolymerisation. Darüber hinaus enthalten die Dentalwerkstoffe vorzugsweise auch organischen und/oder anorganischen Füllstoff.

Als polymerisierbare organische Bindemittel eignen sich alle für einen Dentalwerkstoff brauchbaren Bindemittel, insbesondere monofuktionelle oder polyfunktionelle Methacrylate, die allein oder in Mischungen eingesetzt werden können. Als Bindemittel kommen beispielsweise Methylmethacrylat, Isobutylmethacrylat, Cyclohexylmethacrylat, Tetraethylenglycoldimethacrylat, Triethylenglycoldimethacrylat, Diethylenglycoldimethacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, 2,2-Bis-[4-(2-Hydroxy-3-methacryloxypropoxy)-phenyl]-propan (Bis-GMA) sowie die Reaktionsprodukte aus Isocyanaten, insbesondere Di- und/oder Triisocyanaten und OH-gruppenhaltigen Methacrylaten in Frage. Beispiele dafür sind die Umsetzungsprodukte von 1 Mol Hexamethylendiisocyanat mit 2 Mol 2-Hydroxyethylenmethacrylat, von 1 Mol Tri-(6-isocyanatohexyl)biuret mit 3 Mol 2-Hydroxyethylmethacrylat und von 1 Mol 2,2,4-Trimethylhexamethylendiisocyanat mit 2 Mol 2-Hydroxyethylmethacrylat, die im folgenden als Urethandimethacrylate bezeichnet werden. Der Anteil dieser meist langkettigen Verbindungen im Dentalwerkstoff bewegt sich zwischen 10 und 80 Gew.-%.

Besonders bevorzugt sind 2,2-Bis-[4-(2-Hydroxy-3-methacryloxypropoxy)-phenyl]-propan (Bisphenol-A-Diglycidyldimethacrylat, Bis-GMA), 7,7,9-Trimethyl-4,13-dioxo-3,14-dioxa-5,12-diazahexadecan-1,16-dioxy-dimethacrylat, Triethylenglykoldimethacrylat, 1,10-Decandioldimethacrylat und Mischungen dieser Monomere.

Als Initiatoren für die heißhärtenden Systeme sind Peroxide, insbesondere t-Butylperoxid, Dibenzoylperoxid, Dilauroylperoxid, tert.-Butylperoctat und tert.-Butylperbenzoat bevorzugt. Darüber hinaus sind auch 2,2'-Azoisobuttersäurenitril (AIBN), Benzpinakol und 2,2'-Dialkylbenzpinakole geeignet. Heißhärtende Dentalmaterialien eignen sich besonders zur Herstellung von Inlays und Onlays.

Als Initiatoren für die Kaltpolymerisation werden Radikale liefernde Systeme, zum Beispiel Benzoylperoxid, Lauroylperoxid oder vorzugsweise Dibenzoylperoxid, zusammen mit Aminen wie N,N-Dimethyl-p-toluidin, N,N-Dihydroxyethyl-p-toluidin oder anderen strukturverwandten Aminen eingesetzt.

Amin und Peroxyd werden üblicherweise auf zwei unterschiedliche Komponenten des Dentalwerkstoffs verteilt. Beim Mischen der aminhaltigen Basenpaste mit der peroxidhaltigen Initiatorpaste wird durch die Reaktion von Amin und Peroxid die radikalische Polymerisation initiiert.

Als Initiatoren für die Photopolymerisation können zum Beispiel Benzophenon und seine Derivate sowie Benzoin und seine Derivate verwendet werden. Weitere bevorzugte Photoinitiatoren sind die α-Diketone wie 9,10-Phenanthrenchinon, Diacetyl, Furil, Anisil, 4,4'-Dichlorbenzil, 4,4'-Dialkoxybenzil, Phenylpropandion und Acylphosphinoxide. Besonders bevorzugt wird Campherchinon verwendet. Die Photopolymerisation wird bevorzugt durch Bestrahlen mit Licht in einem Wellenlängenbereich von 400 bis 500 nm initiiert.

Als Initiatoren für dual härtbare Systeme eignen sich Kombinationen aus Kalt- und Photoinitiatoren. Beispielsweise kann die Basenpaste zusätzlich einen Photoinitiator enthalten, so daß die Basenpaste entweder allein als lichthärtender oder zusammen mit der Initiatorpaste als licht- und selbsthärtender Dentalwerkstoff eingesetzt werden kann. Bevorzugt ist die Verwendung von Campherchinon und Dibenzoylperoxid in Kombination mit den oben genannten Aminen.

Dentalmaterialien, die einen Initiator für die Kaltpolymerisation und insbesondere für die Photopolymerisation enthalten, sind bevorzugt.

Als Füllstoffkomponenten eignen sich insbesondere amorphe, kugelförmige Materialien auf der Basis von Mischoxiden aus SiO₂, ZrO₂ und/oder TiO₂ mit einer mittleren durchschnittlichen Partikelgröße von 0,005 bis 2,0 µm, vorzugsweise von 0,1 bis 1 µm, wie sie beispielsweise in der DE-PS 32 47 800 offenbart werden, mikrofeine Füllstoffe, wie pyrogene Kieselsäure oder Fällungskieselsäure sowie Makro- oder Mini-Füllstoffe, wie Quarz-, Glaskeramik- oder Glaspulver, Bariumsilikatgläser, Bariumfluorsilikatgläser und Li/Al Silikatgläser, Bariumgläser, die Oxide von Aluminium oder Silicium, mit einer durchschnittlichen Teilchengröße von 0,01 bis 20 µm, vorzugsweise 0,5 bis 5 µm, sowie röntgenopake Füllstoffe. Unter Mini-Füllstoffen werden Füllstoffe mit einer Partikelgröße von 0,5 bis 1,5 µm und unter Makro-Füllstoffen Füllstoffe mit einer Partikelgröße von 10 bis 20 µm verstanden.

Geeignete röntgenopake Füllstoffe, deren mittlere Partikelgröße 5,0 µm nicht übersteigen sollte, werden in der DE-OS 35 02 594 beschrieben. Besonders bevorzugt ist Ytterbiumtrifluorid.

Vorzugsweise wird als Füllstoff eine Mischung aus (A) amorphen, kugelförmigen Teilchen aus Siliciumdioxid und bis zu 20 Mol-% eines Oxids mindestens eines Elements der Gruppen I, II, III und IV des Periodensystems mit einem Brechungsindex von 1,50 bis 1,58 und mit einer durchschnittlichen Primärteilchengröße von 0,1 bis 1,0 µm, und (B) Quarz-, Glaskeramik- oder Glaspulver oder deren Mischungen mit einem Brechungsindex von 1,50 bis 1,58 und mit einer durchschnittlichen Teilchengröße von 0,5 bis 5,0 µm verwendet.

Der anorganische Füllstoff (A) enthält als Oxid eines Metalls der Gruppen I, II, III und IV des Periodensystems vorzugsweise Strontium- und/oder Zirkonoxid. Die durchschnittliche Primärteilchengröße liegt vorzugsweise im Bereich 0,15 bis 0,5 µm, und der Brechungsindex des anorganischen Füllstoffs (A) vorzugsweise zwischen 1,52 und 1,56. Ein besonders bevorzugter Wert ist 1,53 ± 0,01. Füllstoffe des Typs (A) sind in der DE-PS 32 47 800 beschrieben. Der Füllstoff des Typs (A) kann auch gesintert als Mischung von Agglomeraten mit einer durchschnittlichen Teilchengröße von 1 bis 30 µm vorliegen.

Die durchschnittliche Primärteilchengröße des anorganischen Füllstoffs (B) liegt vorzugsweise zwischen 1,0 und 2,0 µm und besonders bevorzugt zwischen 1,0 und 1,5 µm, während der Brechungsindex Werte vorzugsweise zwischen 1,52 und 1,56 aufweisen soll. Es können auch Füllstoffgemische eingesetzt werden. Bevorzugt werden erfindungsgemäß Ba-Silikatgläser mit einer mittleren Korngröße im Bereich von 1,1 bis 1,3 µm, sowie Sr-Silikatgläser mit einer mittleren Korngröße im Bereich von 1,1 bis 1,3 µm, sowie Li/Al-Silikatgläser mit einer mittleren Korngröße von 1,0 bis 1,6 µm verwendet. Solche Pulver lassen sich z.B. durch Feinmahlung mit einer RS-Ultrafeinstmühle der Firma Reimbold & Strich, Köln, erhalten.

Gegebenenfalls kann die Mischung der Füllstoffe (A) und (B) noch weitere Füllstoffe (C) zur Erzielung einer erhöhten Röntgenopazität und/oder Füllstoffe (D) zur Einstellung der Viskosität enthalten. Als Füllstoff (D) ist mikrofeine, pyrogene oder naß gefällte Kieselsäure bevorzugt. Die Menge an Füllstoff (D) beträgt höchstens 5 Gew.-%, bezogen auf den Dentalwerkstoff.

Weitere bevorzugte Füllstoffe werden in der DE 40 29 230 beschrieben.

Die anorganischen Füllstoffe sind vorzugsweise silanisiert. Als Haftvermittler eignen sich besonders α- und γ-Methacryloxypropyltrimethoxysilan und ähnliche an sich bekannte Stoffe.

Dem Dentalwerkstoff können ferner feinteilige Splitter- oder Perlpolymerisate einverleibt werden, die Homo- oder Copolymere der schon beschriebenen Monomere sein können. Diese Homo- bzw. Copolymeren können ihrerseits mit den beschriebenen anorganischen Füllstoffen, auch röntgenopaken, gefüllt sein. Ferner kann der Dentalwerkstoff die üblichen Pigmentierungsmittel und Stabilisatoren enthalten.

Die erfindungsgemäß bevorzugten thermochromen Farbstoffe sind mit den unterschiedlichsten Dentalwerkstoffen verträglich und erweisen sich besonders bei der Einarbeitung in farblose oder zahnfarbene Dentalwerkstoffe als vorteilhaft, da sie im wesentlichen zu keiner sichtbaren Verfärbung des Materials führen. Die Dentalmaterialien zeichnen sich durch eine dauerhafte Reversibilität des Farbumschlags aus, die durch die Härtung der Materialien nicht beeinträchtigt wird. Zudem läßt sich der Farbumschlag ohne die Gefahr der vorzeitigen Härtung induzieren.

Dentalwerkstoffe im Sinne der Erfindung sind insbesondere Versiegler, permanente und provisorische Füllungsmaterialien, Zemente für Inlays, Onlays, Kronen und Brücken, insbesondere Zemente für die Kieferorthopädie (KFO-Zemente), d.h. Zemente zur Befestigung von Brackets und Zahnklammern, Lacke, wie beispielsweise Fluoridlacke, Stumpfaufbaumaterialien, Plaqueindikatoren und Adhäsive. Adhäsive sind in der Regel ungefüllt während Zemente in der Regel Füllstoff enthalten. Adhäsive wirken als Haftvermittler zwischen Zahnhartsubstanz und Restauration und können in Kombination mit einem Zement eingesetzt werden. Wasserfreie Dentalwerkstoffe und insbesondere lösungsmittelfreie Dentalwerkstoffe sind bevorzugt.

Aufgrund des Gehalts an thermochromen Farbstoff kann der Zahnarzt bei Versieglern, Adhäsiven und Lacken einen vollflächigen Auftrag sicherstellen und bei Füllungsmaterialien und Zementen Materialreste sichtbar machen und sicher zwischen Dentalwerkstoff und natürlicher Zahnsubstanz unterscheiden.

Die erfindungsgemäßen Dentalwerkstoffe enthalten vorzugsweise mehr als 10 Gew.-%, besonders bevorzugt mehr als 20 Gew.-% polymerisierbares Monomer. Die Füllstoffgehalt liegt vorzugsweise im Bereich von 10 bis 90 Gew.-%. Die Dentalwerkstoffe werden hergestellt, indem man Farbstoff, Polymerisationsinitiator und ggf. Füllstoff und Additive in das Bindemittel einarbeitet bis eine homogene Mischung der Bestandteile erhalten wird.

Ganz besonders bevorzugt sind Materialien folgender Zusammensetzung:
(a) > 10 bis 99,98 Gew.-%, vorzugsweise > 20 bis 90 Gew.-% polymerisierbares Monomer; und/oder
(b) 0,01 bis 2 Gew.-%, vorzugsweise 0,1 bis 0,5 Gew.-% thermochromer Farbstoff; und/oder
(c) 0,01 bis 5 Gew.-%, vorzugsweise 0,1 bis 3 Gew.-% Polymerisationsinitiator; und/oder
(d) 0 bis < 89,98 Gew.-%, vorzugsweise 10 bis 80 Gew.-% und ganz besonders bevorzugt 20 bis 70 Gew.-% Füllstoff; und/oder
(e) ggf. 0,01 bis 5 Gew.-% weitere Additive.

Unter Additiven werden übliche Zuschlagstoffe, wie Stabilisatoren, UV-Absorber, Polymerisationsinhibitoren, Farbstoffe, Pigmente und Gleitmittel verstanden.

Der Füllstoffgehalt wird maßgeblich durch die gewünschte Verwendung des Dentalwerkstoffs bestimmt. Zur Verwendung als Adhäsiv sind füllstofffreie Materialien bevorzugt, Füllungsmaterialien weisen vorzugweise einen Füllstoffgehalt von 60 bis 85 Gew.-% und Zemente von 50 bis 80 Gew.-% auf.

Im folgenden wird die Erfindung anhand von Beispielen näher erläutert. Die in den Beispielen eingetzten Farbstoffe enthalten als thermochromen Farbstoff entweder eine säurereaktive Komponente und eine Säurekomponente oder einen Elektronendonator und einen Elektoronenakzeptor. Die Mengenangaben der Beispiele erfolgen, wenn nicht anders angegeben, in Gewichtsprozent.

### Beispiel 1

### Lichthärtendes Füllungsmaterial

Zur Herstellung eines lichthärtenden Füllungsmaterials wurden 0,2 % (diese und die folgenden Prozentangaben beziehen sich, wenn nicht anders angegeben, auf die Gesamtmasse des Dentalmaterials) Chromazone Red (thermochromer Farbstoff; Fa. Davis Liquid Crystals; die farbgebenden Eigenschaften der Farbstoffe basieren auf Elektronendonator/Elektoronenakzeptor-Wechselwirkungen) mittels eines Dispergators in 21,1 % einer Monomermischung der unten angegebenen Zusammensetzung dispergiert. Diese Mischung wurde anschließend mit 51,7% Bariumsilikatglaspulver, 5 % Bariumfluorsilikatglaspulver, 5 % pyrogener Kieselsäure und 17% Ytterbiumtrifluorid zu einem homogenen Composite verarbeitet. Dieses wurde durch dreiminütiges Bestrahlen mit Licht einer Wellenlänge von 400 bis 500 nm gehärtet. Das gehärtete Material zeigte bei 29 °C einen Farbumschlag, bei Temperaturen unterhalb von 29 °C war es rot gefärbt und nach Erhöhung der Temperatur auf über 29 °C verschwand die rote Färbung und das Material wies wieder seine Eigenfarbe auf. Dieser Vorgang ließ sich beliebig oft wiederholen.

| Monomermischung | |
|---|---|
| Bis-GMA | 42,1% |
| 7,7,9-Trimethyl-4,13-dioxo-3,14-dioxa-5,12-diazahexadecan-1,16-dioxy-dimethacrylat (Diurethandimethacrylat) | 37% |
| Triethylenglycoldimethacrylat | 20% |
| Campherchinon | 0,3% |
| Hydrochinonmonoethylether | 0,1% |
| Ethyl-4-dimethylamino-benzoat | 0,5 % |

Die Prozentangaben beziehen sich auf die Masse der Monomermischung.

### Beispiel 2

### Lichthärtendes Füllungsmaterial

Das Beispiel 1 wurde unter Verwendung des thermochromen Farbstoffs Chromazone Blue^{R} (Fa. Davis Liquid Crystals) wiederholt. Die Komponenten wurden auf einem Walzenstuhl miteinander gemischt und das Composite wie in Beispiel 1 beschrieben gehärtet. Das gehärtete Material zeigte bei 29 °C einen reversiblen Farbumschlag, bei Temperaturen unterhalb von 29 °C war es blau gefärbt und bei Temperaturen oberhalb von 29 °C zahnfarben.

### Beispiel 3

### Dualhärtendes Füllungsmaterial

Zur Herstellung eines dualhärtenden Composites bestehend aus Initiator- und Basenpaste wurden für die Basenpaste zunächst 0,05% PSD-O (Orange-Pigment der Shin-Nisso Kako K.K.) in 26,45% Monomermischung der unten angegebenen Zusammensetzung dispergiert. In diese Dispersion wurden dann 43,5% silanisiertes Bariumsilikatglaspulver, 5 % Bariumfluorsilikatglaspulver und 25% Ytterbiumtrifluorid eingearbeitet und eine homogene Paste hergestellt.

| Monomermischung der Basenpaste | |
|---|---|
| Bis-GMA | 49,2% |
| 7,7,9-Trimethyl-4,13-dioxo-3,14-dioxa-5,12-diazahexadecan-1,16-dioxy-dimethacrylat (Diurethandimethacrylat) | 24,8% |
| Triethylenglycoldimethacrylat | 24,7 |
| Campherchinon | 0,3% |
| Ethyl-4-dimethylaminobenzoat | 0,4% |
| 2,6-Di-tert.-butyl-p-kresol | 0,1% |
| N,N-3,5-Di-tert.-butylanilin | 0,5% |

Die Prozentangaben beziehen sich auf die Masse der Monomermischung.

Zur Herstellung der Initiatorpaste wurden 0,05 PSD-O in 28,75% Monomermischung der unten angegebenen Zusammensetzung dispergiert. Anschließend wurde die Dispersion mit 46,2% silanisiertem Bariumsilikatglaspulver und 25% Ytterbiumtrifluorid zu einer homogenen Paste verarbeitet.

| Monomermischung der Initiatorpaste | |
|---|---|
| Bisphenol-A-Diglycidyldimethacrylat (Bis-GMA) | 50,0% |
| 7,7,9-Trimethyl-4,13-dioxo-3,14-dioxa-5,12-diazahexadecan-1,16-dioxy-dimethacrylat (Diurethandimethacrylat) | 24,2% |
| Triethylenglycoldimethacrylat | 24,2% |
| Benzoylperoxid | 1,5% |
| 2,6-Di-tert.-butyl-p-kresol | 0,1% |

Die Prozentangaben beziehen sich auf die Masse der Monomermischung.

Nach dem Mischen gleicher Anteile an Basenpaste und Initiatorpaste härtete die Mischung innerhalb von drei Minuten bei Raumtemperatur vollständig aus. Das gehärtete Material zeigte bei 30 °C einen reversiblen Farbumschlag, unterhalb dieser Temperatur war es orange gefärbt, bei höheren Temperaturen zeigte es seine weißliche Eigenfarbe.

### Beispiel 4

### Dentales Versiegelungsmaterial

Zur Herstellung eines Versiegelungsmaterials wurden 0,8% mikroverkapselter thermochromer Farbstoff Chromicolor^{R} Fast Blue S-17 (Mischung einer säurereaktiven chromogenen Substanz und einer phenolischen Säure; Hersteller Matsui Shikiso Chemical Co.) mittels eines Dispergators in einer Mischung aus 59,2% Bisphenol-A-Diglycidyldimethacrylat (Bis-GMA), 39,2% Triethylenglycoldimethacrylat, 0,3% Campherchinon, 0,1% Hydrochinonmonoethylether und 0,4% Ethyl-(4-dimethylamino)-benzoat dispergiert. Die Aushärtung erfolgte durch Belichten mit Blaulicht in einem Wellenlängenbereich von 400 bis 500 nm für 3 Minuten. Das Material zeigt einen reversiblen Farbumschlag bei 25 °C, unterhalb dieser Temperatur ist es blau gefärbt, darüber praktisch farblos. Das Material ist besonders zur Verwendung als Fissurenversiegler geeignet.

### Beispiel 5

### Dentaler KFO-Zement

Zur Herstellung eines KFO-Zements wurden 1,0% des mikroverkapselten thermochromen Farbstoffs Chromicolor Yellow S-17 (thermochrome säurereaktive Komponente mit einer phenolischen Säurekomponente; Hersteller Matsui Shikiso Chemical Co.) auf einem Walzenstuhls in 87,8% der unten näher beschriebenen Monomermischung eingearbeitet. Anschließend wurde die Mischung mit 11,2% silanisiertem Bariumglaspulver versetzt.

| Monomermischung | |
|---|---|
| Bis-GMA | 64,9% |
| 7,7,9-Trimethyl-4,13-dioxo-3,14-dioxa-5,12-diazahexadecan-1,16-dioxy-dimethacrylat (Diurethandimethacrylat) | 19,8% |
| 1,10-Decandioldimethacrylat | 14,9% |
| Campherchinon | 0,3% |
| 2,6-Di-tert.-butyl-p-kresol | 0,1% |

Die Prozentangaben beziehen sich auf die Masse der Monomermischung.

Die Härtung des Zements erfolgte mittels Blaulicht bei 470 nm durch Belichten für 40 Sekunden. Der Zement wird zur Befestigung von Brackets auf den Zahn aufgetragen und durch Belichten mittels Blaulicht im Wellenlängenbereich von 400 bis 500 nm für 40 Sekunden gehärtet. Das Material zeigt einen reversiblen Farbumschlag bei 20 °C, unterhalb dieser Temperatur ist es gelb gefärbt, darüber weist es seine Eigenfärbung auf.

## Patentansprüche

1. Dentalwerkstoff enthaltend mindestens ein polymerisierbares ethylenisch ungesättigtes Monomer und mindestens einen Initiator für die Kalt-, Heiß- und/oder Photopolymerisation, **dadurch gekennzeichnet, daß** er zusätzlich mindestens einen thermochromen Farbstoff enthält, der in Abhängigkeit von der Temperatur seine Farbe reversibel ändert.

2. Dentalwerkstoff nach Anspruch 1, **dadurch gekennzeichnet, daß** er als Monomer ein monofunktionelles und/oder polyfunktionelles Methacrylat enthält.

3. Dentalwerkstoff nach Anspruch 1 oder 2, **dadurch gekennzeichnet, daß** er mehr als 10 Gew.-% Monomer enthält.

4. Dentalwerkstoff nach einem der Ansprüche 1 bis 3, **dadurch gekennzeichnet, daß** er 0,01 bis 2 Gew.-% thermochromen Farbstoff enthält.

5. Dentalwerkstoff nach einem der Ansprüche 1 bis 4, **dadurch gekennzeichnet, daß** er einen thermochromen Farbstoff enthält, der bei Körpertemperatur farblos ist und sich beim Abkühlen reversibel verfärbt.

6. Dentalwerkstoff nach Anspruch 5, **dadurch gekennzeichnet, daß** sich der thermochrome Farbstoff bei einer Temperatur von 29°C oder weniger verfärbt.

7. Dentalwerkstoff nach Anspruch 1, **dadurch gekennzeichnet, daß** sich der thermochrome Farbstoff bei einer Temperatur von 40 bis 60°C verfärbt.

8. Dentalwerkstoff nach einem der Ansprüche 1 bis 7, **dadurch gekennzeichnet, daß** der thermochrome Farbstoff einen Elektronendonator und einen Elektronenakzeptor enthält.

9. Dentalwerkstoff nach einem der Ansprüche 1 bis 7, **dadurch gekennzeichnet, daß** der thermochrome Farbstoff eine säurereaktive Komponente und eine Säurekomponente enthält.

10. Dentalwerkstoff nach einem der Ansprüche 1 bis 7, **dadurch gekennzeichnet, daß** der thermochrome Farbstoff ein flüssigkristallines Cholesterinderivat enthält.

11. Dentalwerkstoff nach einem der Ansprüche 1 bis 10, **dadurch gekennzeichnet, daß** er organischen und/oder anorganischen Füllstoff enthält.

12. Dentalwerkstoff nach Anspruch 11, **dadurch gekennzeichnet, daß** er 10 bis 90 Gew.-% Füllstoff enthält.

13. Dentalwerkstoff nach einem der Ansprüche 1 bs 12, **dadurch gekennzeichnet, daß** er
(a) > 10 bis 99,98 Gew.-%, vorzugsweise > 20 bis 90 Gew.-% polymerisierbares Monomer; und/oder
(b) 0,01 bis 2 Gew.-%, vorzugsweise 0,1 bis 0,5 Gew.-% thermochromen Farbstoff; und/oder
(c) 0,01 bis 5 Gew.-%, vorzugsweise 0,1 bis 3 Gew.-% Polymerisationsinitiator; und/oder
(d) 0 bis < 89,98 Gew.-%, vorzugsweise 10 bis 80 Gew.-% und ganz besonders bevorzugt 20 bis 70 Gew.-% Füllstoff; und/oder
(e) ggf. 0,01 bis 5 Gew.-% weitere Additive enthält.

14. Verwendung eines thermochromen Farbstoffs zur Herstellung eines Dentalwerkstoffs gemäß einem der vorhergehenden Ansprüche.

## Claims

1. Dental material comprising at least one polymerisable ethylenically unsaturated monomer and at least one initiator for cold, hot and/or photo polymerisation, **characterised in that** it additionally comprises at least one thermo chromic dye, which undergoes a temperature-dependent reversible colour change.

2. Dental material according to Claim 1 **characterised in that** it comprises a monomer that is a mono-functional and/or a poly-functional methacrylate.

3. Dental material according to Claim 1 or 2 **characterised in that** it comprises more than 10 wt.% monomer.

4. Dental material according to one of Claims 1 to 3 **characterised in that** it comprises from 0.01 to 2 wt.% thermo chromic dye.

5. Dental material according to one of Claims 1 to 4 **characterised in that** it comprises a thermo chromic dye, which is colourless at body temperature and undergoes a reversible change of colour upon cooling.

6. Dental material according to Claim 5 **characterised in that** the thermo chromic dye changes colour at a temperature of 29°C or less.

7. Dental material according to Claim 1 **characterised in that** the thermo chromic dye changes colour at a temperature between 40 and 60°C.

8. Dental material according to one of Claims 1 to 7 **characterised in that** the thermo chromic dye comprises an electron donor and an electron acceptor.

9. Dental material according to one of Claims 1 to 7 **characterised in that** the thermo chromic dye comprises an acid-reactive component and an acidic component.

10. Dental material according to one of Claims 1 to 7 **characterised in that** the thermo chromic dye comprises a liquid-crystalline cholesterol derivative.

11. Dental material according to one of Claims 1 to 10 **characterised in that** it comprises an organic and/or inorganic filler.

12. Dental material according to Claim 11 **characterised in that** it comprises from 10 to 90 wt.% filler.

13. Dental material according to one of Claims 1 to 12 **characterised in that** it comprises:
(a) >10 to 99.98 wt.%, preferably >20 to 30 wt.% polymerisable monomer; and/or
(b) 0.01 to 2 wt.%, preferably 0.1 to 0.5 wt.% thermo chromic dye; and/or
(c) 0.01 to 5 wt.%, preferably 0.1 to 3 wt.% polymerisation initiator; and/or
(d) 0 to <89.98 wt.%, preferably 10 to 80 wt.% and most particularly preferred 20 to 70 wt.% filler; and
(e) optionally 0.01 to 5 wt.% further additives.

14. Use of a thermo chromic dye for the preparation of a dental material according to one of the preceding Claims.

## Revendications

1. Matériau dentaire contenant au moins un monomère polymérisable à insaturation éthylénique et au moins un initiateur pour la polymérisation à froid, la polymérisation à chaud et/ou la photopolymérisation, **caractérisé en ce qu'**il contient en outre au moins un colorant thermochromique, dont la coloration varie de manière réversible en fonction de la température.

2. Matériau dentaire selon la revendication 1, **caractérisé en ce qu'**il contient comme monomère un méthacrylate monofonctionnel et/ou polyfonctionnel.

3. Matériau dentaire selon la revendication 1 ou 2, **caractérisé en ce qu'**il contient plus de 10% en poids de monomère.

4. Matériau dentaire selon l'une quelconque des revendications 1 à 3, **caractérisé en ce qu'**il contient 0,01 à 2% en poids de colorant thermochromique.

5. Matériau dentaire selon l'une quelconque des revendications 1 à 4, **caractérisé en ce qu'**il contient un colorant thermochromique qui est incolore à la température corporelle et change de couleur de manière réversible au refroidissement.

6. Matériau dentaire selon la revendication 5, **caractérisé en ce que** le colorant thermochromique change de couleur à une température de 29°C ou moins.

7. Matériau dentaire selon la revendication 1, **caractérisé en ce que** le colorant thermochromique change de couleur à une température de 40 à 60°C.

8. Matériau dentaire selon l'une quelconque des revendications 1 à 7, **caractérisé en ce que** le colorant thermochromique contient un donneur d'électrons et un accepteur d'électrons.

9. Matériau dentaire selon l'une quelconque des revendications 1 à 7, **caractérisé en ce que** le colorant thermochromique contient un composant réagissant aux acides et un composant acide.

10. Matériau dentaire selon l'une quelconque des revendications 1 à 7, **caractérisé en ce que** le colorant thermochromique contient un dérivé de cholestérol en cristaux liquides.

11. Matériau dentaire selon l'une quelconque des revendications 1 à 10, **caractérisé en ce qu'**il contient une charge organique et/ou inorganique.

12. Matériau dentaire selon la revendication 11, **caractérisé en ce qu'**il contient 10 à 90% en poids de charge.

13. Matériau dentaire selon l'une quelconque des revendications 1 à 12, **caractérisé en ce qu'**il contient :
a. > 10 à 99,98% en poids, de préférence > 20 à 90% en poids de monomère polymérisable; et/ou
b. 0,01 à 2% en poids, de préférence 0,1 à 0,5% en poids de colorant thermochromique; et/ou
c. 0,01 à 5% en poids, de préférence 0,1 à 3% en poids d'initiateur de polymérisation; et/ou
d. 0 à < 89,98% en poids, de préférence 10 à 80% en poids, bien mieux encore 20 à 70% en poids de charge; et/ou
e. éventuellement 0,01 à 5% en poids d'autres additifs.

14. Utilisation d'un colorant thermochromique pour la fabrication d'un matériau dentaire selon l'une des revendications précédentes.
